# EUROPEAN PATENT SPECIFICATION

(11) **EP 2 716 637 B1**
(45) Date of publication and mention of the grant of the patent: **19.07.2017**
(21) Application number: 11866730.2
(22) Date of filing: 12.08.2011
(51) Int. Cl.: C07D 311/86, A61K 31/352, A61K 9/00, A61K 9/19, A61K 9/48, A61K 9/20, A61K 9/22, A61P 3/00

(54) **MANGIFERIN AGLYCONE CRYSTAL FORM I AND PREPARATION METHOD THEREOF**
MANGIFERIN-GLYKON-KRISTALLFORM I UND VERFAHREN ZU IHRER HERSTELLUNG
FORME CRISTALLINE I D'AGLYCONE DE MANGIFÉRINE ET PROCÉDÉ DE PRÉPARATION DE CELLE-CI

(30) Priority: 30.05.2011 CN 201110143291
(43) Date of publication of application: 09.04.2014
(73) Proprietor: KPC Pharmaceuticals, Inc., Kunming, Yunnan 650106 (CN)
(72) Inventor: ZHANG, Wei, Yunnan 650106 (CN); LI, Penghui, Yunnan 650106 (CN); GONG, Yunqi, Yunnan 650106 (CN); GAO, Xiaohui, Yunnan 650106 (CN)
(74) Representative: Herrmann, Uwe
(86) International application number: PCT/CN2011/078360
(87) International publication number: WO 2012/162945

(56) References cited:
- CN-A- 101 367 787
- CN-A- 101 669 934
- CN-A- 102 002 031

## Description

This application claims the priority of China Patent Application No. 201110143291.6, filed with the Patent Office of China on May 30, 2011, titled "MANGIFERIN AGLYCONE CRYSTAL FORM I AND PREPARATION METHOD THEREOF".

### Field of the invention

The present invention refers to the field of medicinal chemistry, especially to a mangiferin aglycone crystal form I and preparation method thereof and pharmaceutically acceptable carrier.

### Background of the invention

Mangiferin aglycone, also named Norathyriol, which chemical name is 9H-Xanthen-9-one, 1,3,6,7-tetrahydroxy-; CAS No.: 3542-72-1; molecular formula: C₁₃H₈O₆, molecular weight 260.2; the chemical name thereof is 1,3,6,7-tetrahydroxyxanthone; the structural formula is shown in Formula I.

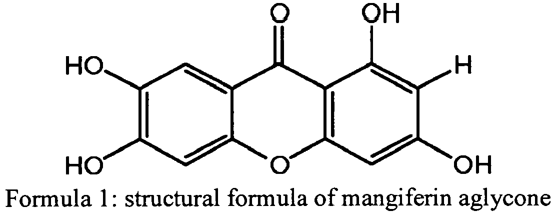

Mangiferin aglycone is mainly found in the following plants: 1. Cystopteris fragilis, the aerial part of Athyrium mesosorum of Athyriaceae; 2. Tripterospermum lanceolatum (Hyata) of Gentianaceae; 3. Hypericum maculatum and Hypericum perforatum Linn. of Hypericaceae; 4. rhizome of Iris nigricans Dinsm. of Iridaceae (yield: 0.00024%); 5. root of Chorophora tinctoria Gaud. of Moraceae.

China Patent "Inhibitory activity of mangiferin aglycone on PTP1B and the use thereof" (Application No.: CN200910184185.5) discloses the inhibitory activity assay of mangiferin aglycone on protein tyrosine phosphatase 1 B, the effect of improving insulin resistance, and the use in a medicament for treating PTP1B-related diseases. The present invention proved that mangiferin aglycone is a competitive inhibitor of recombinant humanized protein tyrosine phosphatase 1B with a IC₅₀ of 9.2 µM by *in vitro* enzyme inhibition assay. Mangiferin aglycone is demonstrated to be able to significantly improve the sensitivity of C57BL/6 normal mice and ob/ob diabetes model mice to insulin, and reduce the blood glucose of the experimental animal by animal experiment. The conclusion shows that mangiferin aglycone is a new type of PTP1B inhibitor, which can be a potential lead compound and can be used in the manufacture of a medicament for treating insulin-resistance-related diabetes and obesity and other metabolic syndromes, tumor and other diseases associated with PTP1B.

In the paper "Studies on the Synthesis and Biological Activities of Mangiferin Derivatives" published by Weifeng Wu, it has been reported that the activities of mangiferin aglycone on some of the targets for diabetes mellitus are superior to that of mangiferin, thus various substituted aglycones are synthesized, and mangiferin aglycone is structurally modified according to the structure combination principles in drug molecular design, and 40 compounds are synthesized in total. Among them, 38 compounds are reported for the first time and are confirmed by HNMR, MS, IR element analysis, etc. Screenings are performed on the synthesized target compounds for their pharmacological activities with various models, and the preliminary results of pharmacological screenings in vitro suggests that such compounds have certain biological activities on DPP-IV target enzyme of diabetes mellitus; and it has been found that 4-sulfonamide compounds have more potent inhibitory activities on cholesterol acyltransferase (ACAT1 and ACAT2); 1,3-hydroxybenzyl derivatives have better inhibitory effects on tumor cells. Structure-activity relationships thereof are investigated by computer-aided drug design and other means, and it is summarized that the introduction of meta-substituted phenylpiperazine can significantly increase the antidiabetic activity.

Synthesis processes of mangiferin aglycone are disclosed in Hayashi T, et al. JP 0782263(1995, 12pp) and Rin T N, et al. JP 04368379(1992, 13pp).

China Patent "Mangiferin aglycone and the preparation and purification methods and application thereof" (Application No.: CN200810196777.4) discloses a method for synthesizing mangiferin aglycone (norathyriol) from mangiferin, the purification thereof, and the use thereof in hypoglycemic agents. This invention has confirmed by animal experiments that mangiferin aglycone can significantly improve IGTT in C57BL/6 normal mouse; meanwhile mangiferin aglycone can increase the *in vivo* secretion of insulin induced by glucose in C57BL/6-normal mouse, suggesting that mangiferin aglycone can be used in the preparation of hypoglycemic agents. This invention also discloses a preparation method of mangiferin aglycone, in which mangiferin is used as starting material, in the presence of phenol and hydroiodic acid hydroiodic acid, carbon-carbon bonds are broken, to obtain mangiferin aglycone crude product, which is then separated and purified by silica gel column chromatography to obtain mangiferin aglycone. The yield of the method is up to 12%, and the purity is over 95%.

Mangiferin aglycone as an active pharmaceutical ingredient is increasingly concerned by scientists. To carry out investigations on physicochemical property, crystal form, in vitro and in vivo release, in vivo metablism, bioavailability and administration form of mangiferin aglycone has great significance on the evaluation of the efficacy of mangiferin aglycone, while the investigations on crystal form of mangiferin aglycone have not yet been reported.

### Summary of the invention

The present invention is made by aiming at the defects of low purity and poor stability of mangiferin aglycone in the prior art, and provides a mangiferin aglycone crystal form I with high purity, high bioavailability and high stability, and the preparation method thereof.

The mangiferin aglycone crystal form I according to the present invention, in which the characteristics of X-ray powder diffraction spectra represented by 2θ angle using Cu-Kα radiation with λ=1.5405A, are as follows.

| 2θ | I/I₀% | 2θ | I/I₀% |
|---|---|---|---|
| 7.32 | 58 | 19.18 | 11 |
| 7.80 | 24 | 25.56 | 16 |
| 9.96 | 19 | 26.42 | 22 |
| 12.46 | 100 | 27.58 | 13 |
| 13.06 | 30 | 28.58 | 19 |
| 15.72 | 16 | | |

The intensity of diffraction peak at 2θ of 12.46 of the X-ray powder diffraction spectrum is 100%.

The infrared absorption spectra of the mangiferin aglycone crystal form I have characteristic absorption peaks at wave numbers at 3347±2, 3146±2, 1652±2, 1616±2, 1506±2, 1418±2, 1373±2, 1304±2, 1225±2, 1127±2, 800±2, 754±2, 732±2, 706±2, 664±2, 619±2, 570±2, 542±2 and 447±2 cm-1.

The thermal transition temperature of DSC analysis of the mangiferin aglycone crystal form I is 384.7∼388.4°C.

The melting decomposition temperature of the mangiferin aglycone crystal form I is 379.1∼403.0°C.

The TGA analysis of mangiferin aglycone crystal form I shows a melting decomposition accompanied with a mass attenuation of 31%∼34%.

The present invention also provides a method preparing the mangiferin aglycone crystal form I, comprising the following steps:

Step 1: A solvent is added to mangiferin aglycone to dissolve it thoroughly, wherein the solvent is a mixture of water and aliphatic alcohol having 1∼4 carbon atoms, or a mixture of water and aliphatic ketone having 1∼4 carbon atoms;

Step 2: 0%∼5% activated charcoal and 0%∼10% acetic acid are added based on the mass of mangiferin aglycone, and filter;

Step 3: The filtrate is allowed to stand, and crystal is slowly precipitated, to obtain the mangiferin aglycone crystal form I.

Preferably, the volume ratio between the aliphatic alcohol having 1∼4 carbon atoms or the aliphatic ketone having 1∼4 carbon atoms and water in the solvent of step 1 is 1:0.01∼1:100, the solvent is preferably a mixture of one or two of aqueous methanol solution, aqueous ethanol solution, and aqueous acetone solution.

More preferably, the mass/volume ratio in g/ml between the mangiferin aglycone and the solvent in step 1 is 1:10∼200.

Preferably, solvent added in step 1 is heated to 50∼100 °C.

Preferably, the temperature for the standing in step 3 is -5∼40°C and kept for 1∼72 hours.

The present invention also provides a pharmaceutical composition, comprising effective amount of the mangiferin aglycone crystal form I and pharmaceutically acceptable carrier, which is formulated into pharmaceutically acceptable dosage forms, such as freeze-dried powder for injection, soft capsule, capsule, sustained-release tablet, dispersible tablet, etc.

In the pharmaceutical composition, the content of mangiferin aglycone crystal form I per dosage unit is 1 mg∼100 mg, preferably 5 mg∼50 mg.

The freeze-dried powder for injection of the pharmaceutical composition provided in the present invention, the freeze-dried powder for injection is consisted by weight of:

| | |
|---|---|
| mangiferin aglycone crystal form I | 1 ∼ 100mg, |
| cosolvent | 1 ∼ 200mg, and |
| excipient | 2 ∼ 1000mg. |

For the freeze-dried powder for injection of the pharmaceutical composition provided in the present invention, the cosolvent is one of water-soluble cyclodextrin or alkaline respectively, wherein the water-soluble cyclodextrin is preferably hydroypropyl-β-cyclodextrin or methyl-β-cyclodextrin, and the alkaline is preferably sodium bicarbonate, sodium carbonate, sodium hydroxide, arginine, lysine or meglumine; the excipient is one selected from the group consisting of mannitol, low molecular dextran, sodium chloride, sucrose and glucose, or any combination thereof.

For the soft capsule of the pharmaceutical composition provided by the present invention, the capsule content is consisted of:

| | |
|---|---|
| mangiferin aglycone crystal form I | 1 mg ∼ 100 mg, and |
| dispersing agent | 100 mg ∼ 1000 mg |

For the capsule of the pharmaceutical composition provided by the present invention, the dispersing agent may be any one of soybean oil, corn oil, peanut oil, linseed oil, fish oil, evening primrose oil, α-linolenic acid, γ-linolenic acid, docosahexaenoic acid (DHA) and eicosapentaenoic acid (EPA).

For the capsule of the pharmaceutical composition provided by the present invention, the capsule shell is consisted of:

| | |
|---|---|
| geletin | 60 mg, |
| glycerol | 20 mg, and |
| ethyl p-hydroxybenzoate | 0.01 mg ∼ 0.3 mg. |

For the sustained-release tablet of the pharmaceutical composition provided by the present invention, the sustained tablet is consisted of:

| | |
|---|---|
| mangiferin aglycone crystal form I | 1 mg ∼ 100 mg, |
| skeleton agent | 10 mg ∼ 1000 mg, |
| filler | 30 mg ∼ 2000 mg, and |
| magnesium stearate | 1 mg ∼ 5 mg. |

For the sustained-release tablet of the pharmaceutical composition provided by the present invention, the skeleton agent is preferably one selected from the group consisting of hydroxypropyl methyl cellulose, stearic acid, ethyl cellulose and beewax, or any combination thereof; the filler is preferably one selected from the group consisting of lactose, pregelatinized starch, microcrystalline cellulose and micro powder silica gel, or any combination thereof.

For the dispersible tablet of the pharmaceutical composition provided by the present invention, the dispersible tablet is consisted of:

| | |
|---|---|
| mangiferin aglycone crystal form I | 1 mg ∼ 100 mg, |
| disintegrating agent | 10 mg ∼ 1000 mg, |
| filler | 30 mg ∼ 2000 mg, and |
| magnesium stearate | 1 mg ∼ 5 mg. |

The dispersible tablet of the pharmaceutical composition provided by the present invention, the disintegrating agent is preferably any one selected from lactose and mannitol, or a mixture thereof; the filler is preferably one selected from the group consisting of microcrystalline cellulose, crosslinked polyvinylpyrrolidone, polyvidone and micro powder silica gel, or any combination thereof.

The X-ray powder diffraction analysis, DSC and TG-DTA analysis, IR and HPLC analysis of the mangiferin aglycone crystal form I of the present invention, and the comparison with the X-ray powder diffraction of mangiferin aglycone crystal in the prior art (reference substance, purchased from SigmaCorporation, Lot No.: 8420-201002) suggests that it is a new crystal form. The mangiferin aglycone crystal form I has high purity, high stability to light, humidity and heat, high stability in alkali solution, and high bioavailability *in vivo.*

The preparation method of the mangiferin aglycone crystal form I according to the present invention is easy to operate, and employs less amount of solvent for crystallization compared with crystallization methods in the prior art, and is easy for industrialized production with low production cost, and the product content is up to 98% or more.

### Description of the drawings

Figure 1: X-ray powder diffraction pattern of mangiferin aglycone crystal form I;
Figure 2: DSC and TG-DTA spectra of mangiferin aglycone crystal form I;
Figure 3: IR spectrum of mangiferin aglycone crystal form I;
Figure 4: HPLC chromatogram of mangiferin aglycone crystal form I;
Figure 5: X-ray powder diffraction pattern of the mangiferin aglycone from Sigma Corporation;
Figure 6: the comparison of drug concentrations in blood at different time points after orally administration of mangiferin aglycone to Beagle dogs.

### Detailed embodiments

To further understand the present invention, the preferred embodiments of the present invention are described below in conjunction with Examples, however, it should be appreciated that these description are only for further illustrating the characteristics and advantages of the present invention, rather than limiting the claims of the present invention.

The effects of the present invention will be illustrated below in detailed Examples, however, the scope of protection of the present invention is not limited by the following Examples.

### Example 1

1 kg product of mangiferin aglycone to be refined was taken, and 200 L 90% aqueous solution of acetone was added, and the mixture was heated under reflux to be thoroughly dissolved, then 50 g activated charcoal was added, and 0.01 L acetic acid was added, and filtered. The filtrate was allowed to stand at 25°C for 72 hours, the crystal was slowly precipitated, filtered, washed, and dried under reduced pressure at 60°C, to obtain 0.75 kg refined product of mangiferin aglycone. The yield of the purifying procedure was 70.5%, and the content was 98.8%.

### Example 2

1 kg product of mangiferin aglycone to be refined was taken, and 50 L 50% aqueous solution of methanol was added, and the mixture was heated under reflux to be thoroughly dissolved, and 0.02 L acetic acid was added, and filtered. The filtrate was allowed to stand at 4°C for 20 hours, the crystal was slowly precipitated, filtered, washed, and dried under reduced pressure at 60°C, to obtain 0.85 kg refined product of mangiferin aglycone. The yield of the purifying procedure was 85%, and the content was 98.2%.

### Example 3

1 kg product of mangiferin aglycone to be refined was taken, and 100 L aqueous solution of absolute ethanol was added, and the mixture was heated under reflux to be thoroughly dissolved, then 100 g activated charcoal was added, and filtered. The filtrate was allowed to stand at -5°C for 1 hour, the crystal was precipitated, filtered, washed, and dried under reduced pressure at 60°C, to obtain 0.9 kg refined product of mangiferin aglycone. The yield of the purifying procedure was 90%, and the content was 98.0%.

### Example 4

1 kg product of mangiferin aglycone to be refined was taken, and 150 L 10% aqueous solution of isopropanol was added, and the mixture was heated under reflux to be thoroughly dissolved, then 20 g activated charcoal was added, and 0.02 L acetic acid was added, and filtered. The filtrate was allowed to stand at 25°C for 36 hours, the crystal was precipitated, filtered, washed, and dried under reduced pressure at 60°C, to obtain 0.78 kg refined product of mangiferin aglycone. The yield of the purifying procedure was 78%, and the content was 98.3%.

### Example 5

1 kg product of mangiferin aglycone to be refined was taken, and 100 L 60% aqueous solution of butanone was added, and the mixture was heated under reflux to be thoroughly dissolved, then 70 g activated charcoal was added, and 0.02 L acetic acid was added, and filtered. The filtrate was allowed to stand at 30°C for 52 hours, the crystal was precipitated, filtered, washed, and dried under reduced pressure at 60°C, to obtain 0.74 kg refined product of mangiferin aglycone. The yield of the purifying procedure was 74%, and the content of mangiferin aglycone in the refined product of mangiferin aglycone was 98.7%.

### Example 6: The purity assay of mangiferin aglycone in the mangiferin aglycone crystal form I according to the present invention

The instrument used was Agilent 1200 HPLC, comprising G1311A quaternary gradient pump, G1322A vacuum degasser, G1329A automatic sampler, G1315D UV detector, G1316A column oven, Agilent 1200 ChemStation, and the chromatographic column was Agilent SB-C18 150×4.6mm.

Octadecylsilane-bonded silica gel was used as filler, and a mixed solution of methanol-acetonitrile-0.2% aqueous solution of phosphoric acid was used as mobile phase. In the mixed solution, the volume ratio of methanol-acetonitrile-0.2% aqueous solution of phosphoric acid was 10:30:60, the detection wavelength was 258 nm, and the theoretical plate number for the peak of mangiferin aglycone was calculated to be 5000.

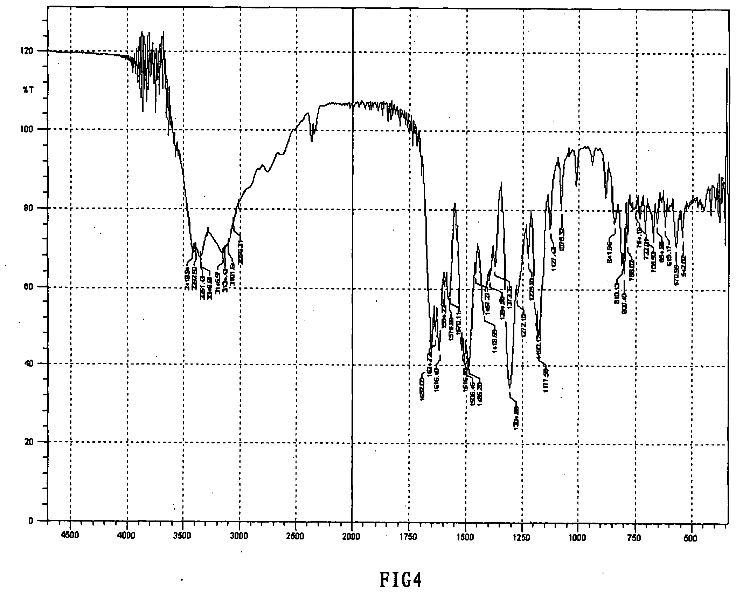

The refined products of mangiferin aglycone prepared in Examples 1, 2, 3, 4 and 5 were taken as test samples, and methanol was added to dissolve and dilute the examples, to obtain a solution with a concentration of 0.2 mg/ml as test sample solution, and 5 µl of the test sample solution was taken and injected into the liquid chromatograph, to record the chromatogram, as shown in Figure 4, and the result of chromatographic analysis is shown in Table 1:

**Table 1 the result of chromatographic analysis of the test solution of mangiferin aglycone**

| Peak No. | Retention time (min) | Area | Area percentage (%) | Peak height |
|---|---|---|---|---|
| 1 | 5.372 | 448.97549 | 100.00 | 58.0842 |

Mangiferin aglycone reference substance (content 100%) purchased from Sigma Corporation was taken, methanol was added to dissolve mangiferin aglycone and dilute the same, to obtain a solution with a concentration of 0.2mg/ml as reference substance solution. 5 µl of the reference substance solution was taken and injected into the liquid chromatograph, to record the chromatogram.

The content was calculated using single point external standard method, the content of the test sample was obtained by calculating the ratio between the peak area of the test solution and the peak area of the reference substance solution. The results of the test samples of Examples 1∼5 (with respect to the weight ratio of mangiferin aglycone) were 98.8%, 98.2%, 99.0%, 98.3%, 98.7%, respectively, based on dry products.

### Example 7: The comparative analysis of X-ray diffraction of the mangiferin aglycone crystal form I according to the present invention and the mangiferin aglycone in the prior art

instrument: D/MAX-2200 diffractometer, Rigaku, Japan
target: Cu-Kα radiation (λ=1.5405A), 2θ=2°∼70°
step angle: 0.04°
tube voltage: 36KV
tube current: 30 mA
scanning speed: 10°/min
filter disc: graphite monochromator

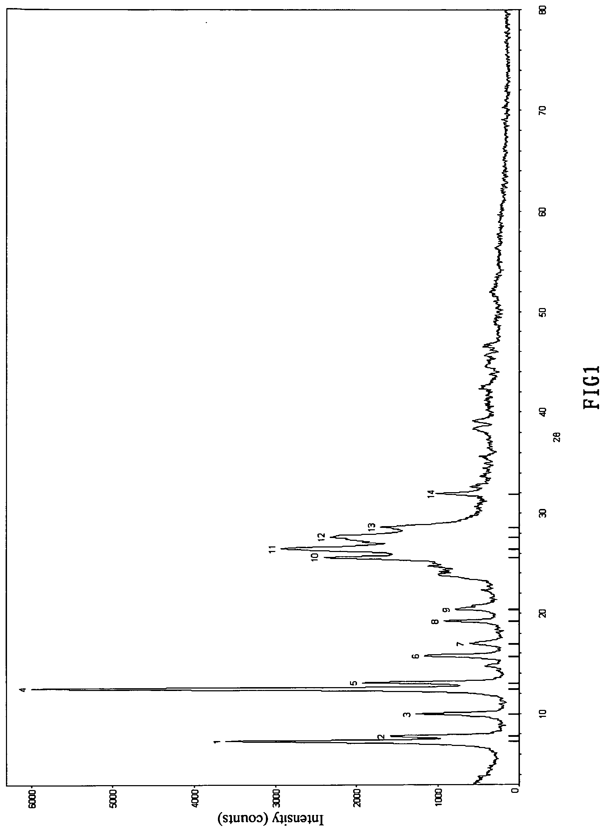

The X-ray powder diffraction of mangiferin aglycone crystal form I prepared in Examples 1∼5 represented by angle 2θ using Cu-Kα radiation with λ=1.5405A is shown in Figure 1, the spectral characteristics are as follows:

| 2θ | I/I₀% | 2θ | I/I₀% |
|---|---|---|---|
| 7.32 | 58 | 19.18 | 11 |
| 7.80 | 24 | 25.56 | 16 |
| 9.96 | 19 | 26.42 | 22 |
| 12.46 | 100 | 27.58 | 13 |
| 13.06 | 30 | 28.58 | 19 |
| 15.72 | 16 | | |

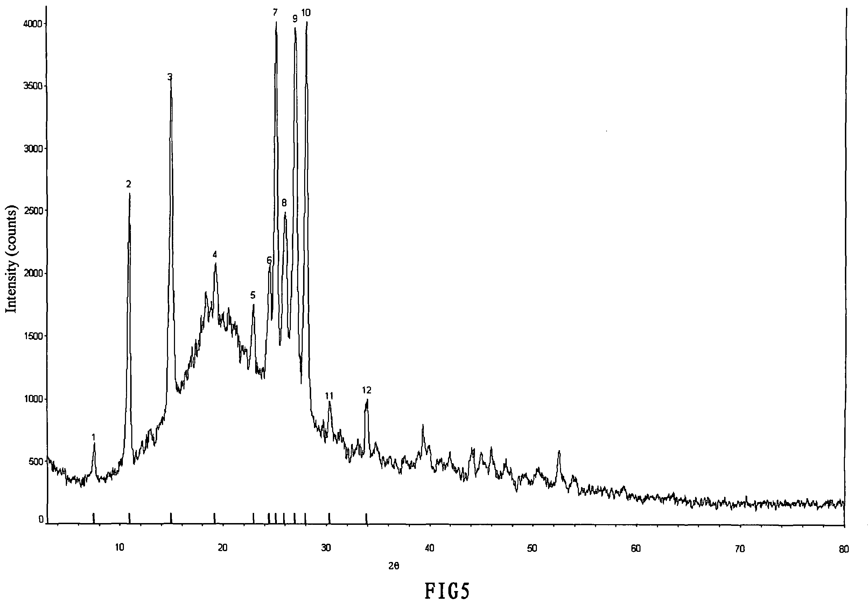

The X-ray powder diffraction of the mangiferin aglycone crystal in the prior art (reference substance of mangiferin aglycone, purchased from Sigma Corp., Batch No. 8420-201002) represented by 2θ angle using Cu-Kα radiation with λ=1.5405A is shown in Figure 5, the spectral characteristics are as follows:

| 2θ | I/I₀% | 2θ | I/I₀% |
|---|---|---|---|
| 7.52 | 10 | 25.12 | 87 |
| 10.92 | 73 | 25.94 | 34 |
| 14.96 | 86 | 26.96 | 88 |
| 19.24 | 16 | 28.02 | 100 |
| | | | |
| 22.96 | 18 | 33.92 | 15 |
| 24.50 | 25 | | |

The above analysis results show that the intensity of the diffraction peak at 2θ of 28.02 of X-ray powder diffraction of the mangiferin aglycone crystal in the prior art is 100%, while the intensity of the diffraction peak at 2θ of 12.46 of X-ray powder diffraction of the mangiferin aglycone crystal form I according to the present invention is 100%, suggesting it is a new crystal form.

### Example 8: The differential scanning calorimetry (DSC) analysis of the mangiferin aglycone crystal form I according to the present invention

instrument: NETZSCH STA 409 PG/PC
range: 35-350°C
heating rate: 5°C/min

The DSC thermal transition temperature of the mangiferin aglycone crystal form I prepared in Examples 1-5 was 124∼128°C, and the melting decomposition temperature was 370-372°C.

### Example 9: The thermogravimetry-differential thermal (TGA) analysis

instrument: NETZSCH STA 409 PG/PC
TG range: 5 mg
DTA range: ±250µV
reference substance: Al₂O₃
temperature range: 35∼350°C
heating rate: 5°C/min

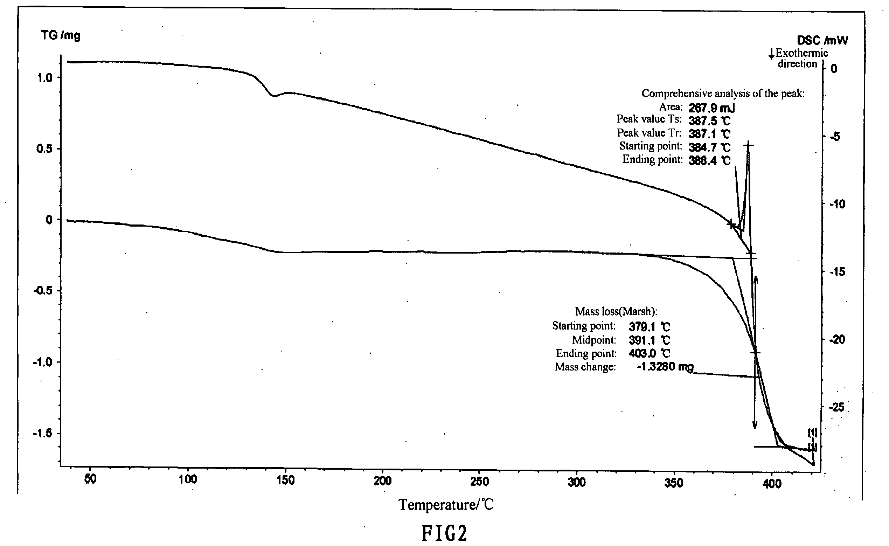

The result shows that the melting decomposition temperature of the mangiferin aglycone crystal form I prepared in Examples 1∼5 was 370∼372°C, accompanied by 12%∼15% of mass attenuation, as shown in Figure 2.

### Example 10: Infrared spectrum (IR) analysis

Instrument: Shimadzu FTIR-8400S infrared spectrometer

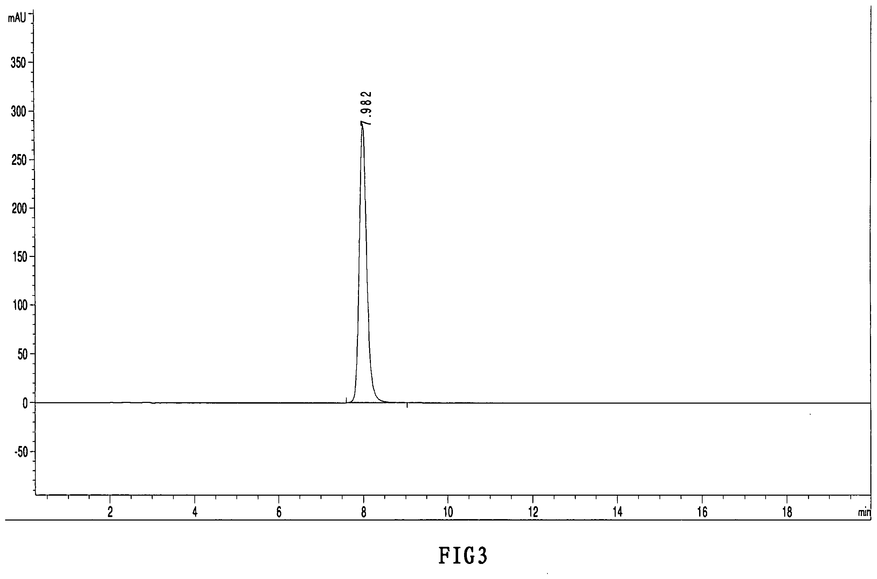

The IR wave numbers (cm⁻¹) of the mangiferin aglycone crystal form I prepared in the Examples 1∼5 (potassium bromide pellet) are shown in Figure 3:
3347, 3146, 1652, 1616, 1506, 1418, 1373, 1304, 1225, 1127, 800, 754, 732, 706, 664, 619, 570, 542 and 447.

### Example 11: The stability of mangiferin aglycone crystal form I

Active destructing test of mangiferin aglycone crystal form I: high purity mangiferin aglycone obtained in the same batch was respectively subjected to the following treatments:
(1) Destruction by strong acid: 0.5 mg sample was precisely weighed in a 50 ml measuring flask, and 1 ml of 0.1 mol/L hydrochloric acid aqueous solution was added, mixed evenly, and was allowed to stand for 48 hours at 20∼30°C;
(2) Destruction by strong base: 0.5 mg sample was precisely weighed in a 50 ml measuring flask, and 1 ml of 0.1 mol/L sodium hydroxide aqueous solution was added, mixed evenly, and was allowed to stand for 48 hours at 20∼30°C;
(3) Destruction by strong oxidant: 0.5 mg sample was precisely weighed in a 50 ml measuring flask, and 1 ml of 10% aqueous solution of hydrogen peroxide (H₂O₂) was added, mixed evenly, and was allowed to stand for 48 hours at 20∼30°C;
(4) Destruction by high temperature: 0.5 mg sample was precisely weighed in a 50 ml measuring flask, and was allowed to stand for 48 hours at 100°C;
(5) Destruction by strong light: 0.5 mg sample was precisely weighed in a 50 ml measuring flask, and was allowed to stand for 240 hours under strong light condition of 4500±500 lux. The above-mentioned testing samples were taken out at specified time points, and methanol was added to dissolve and diluted the sample to the scale mark, shook well, then undestructed samples were analyzed by HPLC.

Results are shown in Table 2:

**Table 2. Stability test of mangiferin aglycone crystal form I**

| test condition | peak area of mangiferin aglycone | situations of other degradation products |
|---|---|---|
| destructing by strong acid | 234 | No new peak for degradation product was found |
| destructing by strong base | 0 | UV shift occurred, and there were many peaks for degradation products |
| destructing by high temperature | 233 | No new peak for degradation product was found |
| destructing by strong oxidant | 215 | One new degradation product was found |
| destructing by strong light | 234 | No peak for degradation product was found |
| undestructed | 234 | No new peak for degradation product was found |

The above test results show that mangiferin aglycone crystal form I is stable to light, moisture, heat, etc., and is easy to be manufactured and stored.

### Example 12: Comparison of stability between solutions of mangiferin aglycone crystal form I and commercially available mangiferin aglycone

Appropriate amounts of mangiferin aglycone crystal form I and mangiferin aglycone were taken respectively, and phosphate buffer of pH 8.0, pH 9.0 and pH 10.0 were added respectively, metered to 100 ml, and were allowed to stand at 37°C, and samples were taken at 2, 4, 6, 8 and 12 hours respectively, and the content of mangiferin aglycone were measured by HPLC method. Results are shown in Table 3:

**Table 3. Stability of solution of mangiferin aglycone crystal form I**

| | pH8 | | pH9 | | pH10 | |
|---|---|---|---|---|---|---|
| Standing time | Crystal form | Mangiferin aglycone | Crystal form | Mangiferin aglycone | Crystal form | Mangiferin aglycone |
| | I % | % | I % | % | I % | % |
| 2h | 98.6 | 98.5 | 98.5 | 97.3 | 97.2 | 97.7 |
| 4h | 98.4 | 97.5 | 98.2 | 94.3 | 97.4 | 93.8 |
| 6h | 98.3 | 96.3 | 98.0 | 93.4 | 97.0 | 92.1 |
| 8h | 98.2 | 94.5 | 97.8 | 92.3 | 96.3 | 91.0 |
| 12h | 97.5 | 93.2 | 97.2 | 91.0 | 95.0 | 90.3 |

The above table shows that, in the study of stability comparison of high purity mangiferin aglycone crystal form I and mangiferin aglycone in different alkaline phosphate buffer solutions, the stability of solution of mangiferin aglycone crystal form I of high purity is better.

### Example 13: Absorption characteristics and blood concentration characteristics of mangiferin aglycone crystal form I and commercially available mangiferin aglycone in vivo

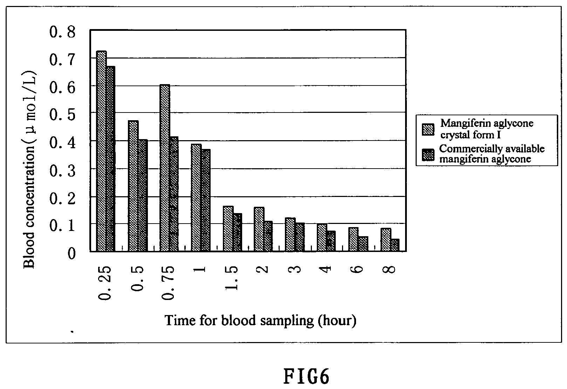

Solid powder of mangiferin aglycone crystal form I and commercially available mangiferin aglycone (reference substance of mangiferin aglycone, purchased from Sigma Corporation, Lot No.: 8420-201002) were administered to Beagle dogs intragastrically respectively. The dosage of administration was 6mg/kg, and arterial blood of the dogs was drawn at different time points after administration to determine the content of mangiferin aglycone (See Table 4 and Figure 6).

**Table 4. Comparison of blood concentrations at different time points after mangiferin aglycone samples were administered orally to Beagle dogs**

| Time (hr) | Mangiferin aglycone concentration in serum(µmol/L) | |
|---|---|---|
| | Mangiferin aglycone crystal form I | Commercially available mangiferin aglycone |
| 0.25 | 0.7212 | 0.6658 |
| 0.5 | 0.4702 | 0.4039 |
| 0.75 | 0.6006 | 0.4141 |
| 1 | 0.3868 | 0.3659 |
| 1.5 | 0.1627 | 0.1363 |
| 2 | 0.1583 | 0.1084 |
| 3 | 0.1187 | 0.1000 |
| 4 | 0.096 | 0.0701 |
| 6 | 0.0844 | 0.0526 |
| 8 | 0.0808 | 0.0413 |

Results show that under the condition of orally taking the same dosage, the blood concentration and the time to reach peak concentration of mangiferin aglycone of different crystal forms were different, wherein the blood concentration of crystal form represented by commercially available mangiferin aglycone was significantly lower than that of mangiferin aglycone crystal form I, i.e. the oral bioavailability of mangiferin aglycone crystal form I was higher.

### Example 14:

Freeze-dried powder for injection of mangiferin aglycone crystal form I: formula for 1000 injections

| | |
|---|---|
| mangiferin aglycone crystal form I | 1 g |
| meglumine | 1 g |
| mannitol | 2 g |

dispensed into 1000 injections, dissolved in water, and freeze-dried to obtain the product.

### Example 15:

Freeze-dried powder for injection of mangiferin aglycone crystal form I: formula for 1000 injections

| | |
|---|---|
| mangiferin aglycone crystal form I | 100 g |
| methyl-β-cyclodextrin | 100 g |
| mannitol | 1000 g |

dispensed into 1000 injections, dissolved in water, and freeze-dried to obtain the product.

### Example 16:

Freeze-dried powder for injection of mangiferin aglycone crystal form I: formula for 1000 injections

| | |
|---|---|
| mangiferin aglycone crystal form I | 50 g |
| hydroxypropyl-β-cyclodextrin | 20 g |
| low molecular dextran | 200 g |

dispensed into 1000 injections, dissolved in water, and freeze-dried to obtain the product.

### Example 17:

Freeze-dried powder for injection of mangiferin aglycone crystal form I: formula for 1000 injections

| | |
|---|---|
| mangiferin aglycone crystal form I | 80 g |
| arginine | 23 g |
| mannitol | 30 g |
| low molecular dextran | 500 g |

dispensed into 1000 injections, dissolved in water, and freeze-dried to obtain the product.

### Example 18:

Freeze-dried powder for injection of mangiferin aglycone crystal form I: formula for 1000 injections

| | |
|---|---|
| mangiferin aglycone crystal form I | 50 g |
| sodium bicarbonate | 10 g |
| sucrose | 100 g |

dispensed into 1000 injections, dissolved in water, and freeze-dried to obtain the product.

### Example 19: Soft capsule of mangiferin aglycone crystal form I

Formula(1000 capsules):

| | |
|---|---|
| contents: mangiferin aglycone crystal form I | 30 g |
| soybean oil | 100 g |
| capsule shell: gelatin | 60.2 g |
| glycerol | 19.8 g |
| water | q.s. |
| ethyl hydroxybenzoate (Ethyl paraben) | 0.12 g |

Preparation method: mangiferin aglycone crystal form I was taken, and added into soybean oil, stirred to be dissolved completely, and soft capsules were compressed, dried, prepared into 1000 capsules, to obtain products of a strength 30 mg.

### Example 20: Soft capsule of mangiferin aglycone crystal form I

Formula(1000 capsules):

| | |
|---|---|
| contents: mangiferin aglycone crystal form I | 60 g |
| corn oil | 200 g |
| capsule shell: gelatin | 60.2 g |
| glycerol | 19.8 g |
| water | q.s. |
| ethyl hydroxybenzoate (Ethyl paraben) | 0.12 g |

Preparation method: mangiferin aglycone crystal form I was taken, and added into soybean oil, stirred to be dissolved completely, and soft capsules were compressed, dried, prepared into 1000 capsules, to obtain products of a strength 200 mg.

### Example 21: Soft capsule of mangiferin aglycone crystal form I

Formula(1000 capsules):

| | |
|---|---|
| Contents: mangiferin aglycone crystal form I | 100 g |
| α-linolenic acid | 1000 g |
| capsule shell: gelatin | 60.2 g |
| glycerol | 19.8 g |
| water | q.s. |
| ethyl hydroxybenzoate (Ethyl paraben) | 0.12 g |

Preparation method: mangiferin aglycone crystal form I was taken, and added into peanut oil, mixed to dissolve completely, and soft capsules were compressed, dried, prepared into 1000 capsules, to obtain products of a strength 100 mg.

### Example 22: Sustained release tablet of mangiferin aglycone crystal form I

Formula (1000 tablets):

| | |
|---|---|
| mangiferin aglycone crystal form I | 60 g |
| hydroxypropyl methyl cellulose | 100 g |
| microcrystalline cellulose | 30 g |
| pregelatinized starch | 30 g |
| micro powder silica gel | 1 g |
| magnesium stearate | 1 g |

Preparation method: Materials were sieved through a 100-mesh screen respectively, and the materials were weighed according to the formula amount, mixed evenly, stirred to prepare dough mess, and granulated through 18∼24-mesh screen, dried in the air at 70°C, compressed, film-coated, to obtain products of a strength 60 mg.

### Example 23: Sustained release tablet of mangiferin aglycone crystal form I

Formula(1000 tablets):

| | |
|---|---|
| mangiferin aglycone crystal form I | 100 g |
| stearic acid | 1000 g |
| lactose | 30 g |
| pregelatinized starch | 30 g |
| micro powder silica gel | 20 g |
| magnesium stearate | 1 g |

Preparation method: Materials were sieved through a 100-mesh screen respectively, and the materials were weighed according to the formula amount, mixed evenly, stirred to prepare dough mess, and granulated through 18∼24-mesh screen, dried in the air at 70°C, compressed, film-coated, to obtain products of a strength 100 mg.

### Example 24: Sustained release tablet of mangiferin aglycone crystal form I

Formula(1000 tablets):

| | |
|---|---|
| mangiferin aglycone crystal form I | 80 g |
| ethyl cellulose | 200 g |
| microcrystalline cellulose | 1500 g |
| pregelatinized starch | 400 g |
| micro powder silica gel | 50 g |
| magnesium stearate | 5 g |

Preparation method: Materials were sieved through a 100-mesh screen respectively, and the materials were weighed according to the amount in the formula, mixed evenly, stirred to prepare dough mess, and granulated through 18∼24-mesh screen, dried in the air at 70°C, compressed, film-coated, to obtain products of a strength 300 mg.

### Example 25: Sustained release tablet of mangiferin aglycone crystal form I

Formula(1000 tablets):

| | |
|---|---|
| mangiferin aglycone crystal form I | 5 g |
| beewax | 1000 g |
| lactose | 1500 g |
| pregelatinized starch | 400 g |
| magnesium stearate | 5 g |

Preparation method: Materials were sieved through a 100-mesh screen respectively, and the materials were weighed according to the amount in the formula, mixed evenly, stirred to prepare dough mess, and granulated through 18∼24-mesh screen, dried in the air at 70°C, compressed, film-coated, to obtain products of a strength 400 mg.

### Example 26: Dispersible tablet of mangiferin aglycone crystal form I

Formula(1000 tablets):

| | |
|---|---|
| mangiferin aglycone crystal form I | 100 g |
| lactose | 100 g |
| microcrystalline cellulose | 250 g |
| crospolyvinylpyrrolidone | 100 g |
| polyvidone | 15 g |
| micro powder silica gel | 16 g |
| magnesium stearate | 3 g |

Starting materials were sieved through a 100-mesh screen respectively, and lactose, microcrystalline cellulose, crospolyvinylpyrrolidone, polyvidone were weighed according to the formula amount and mixed evenly; then mangiferin aglycone was added and mixed evenly; then magnesium stearate, micro powder silica gel were added and mixed evenly, compressed, to obtain mangiferin aglycone dispersible tablet of the present invention.

The products are light yellow tablets. Dispersible uniformity, dissolution and content were tested according to regulations, and the dispersible uniformity conformed with the regulations, the dissolution was 95%, and the content was 98.0%.

### Example 27: Dispersible tablet of mangiferin aglycone crystal form I

Formula(1000 tablets):

| | |
|---|---|
| mangiferin aglycone crystal form I | 20 g |
| lactose | 150 g |
| microcrystalline cellulose | 50 g |
| crospolyvinylpyrrolidone | 50 g |
| polyvidone | 5 g |
| micro powder silica gel | 10 g |
| magnesium stearate | 1 g |

Starting materials were sieved through a 100-mesh screen respectively. Lactose, microcrystalline cellulose, crospolyvinylpyrrolidone and polyvidone were weighed according to the formula amount and mixed evenly; then mangiferin aglycone was added and mixed evenly; then magnesium stearate, micro powder silica gel were added and mixed evenly, compressed, to obtain mangiferin aglycone dispersible tablet of the present invention.

### Example 28: Dispersible tablet of mangiferin aglycone crystal form I

Formula(1000 tablets):

| | |
|---|---|
| mangiferin aglycone crystal form I | 150 g |
| lactose | 150 g |
| mannitol | 150 g |
| crospolyvinylpyrrolidone | 100 g |
| magnesium stearate | 5 g |
| 95% ethanol | q.s. |

Starting materials were sieved through a 100-mesh screen respectively. Mangiferin aglycone, lactose, mannitol and crospolyvinylpyrrolidone were weighed according to the formula amount and mixed evenly; dough mess was prepared using 95% ethanol as wetting agent, granulated through 1-mesh screen, and the wet granules were dried under a condition of 50∼80°C; granule sizing was performed using 20-mesh screen, then magnesium stearate and micro powder silica gel were added and mixed evenly, compressed, to obtain mangiferin aglycone dispersible tablet of the present invention.

### Example 29: Dispersible tablet of mangiferin aglycone crystal form I

Formula(1000 tablets):

| | |
|---|---|
| mangiferin aglycone crystal form I | 75 g |
| lactose | 250 g |
| mannitol | 200 g |
| crospolyvinylpyrrolidone | 100 g |
| magnesium stearate | 10 g |
| 95% ethanol | q.s. |

Starting materials were sieved through a 100-mesh screen respectively. Mangiferin aglycone, lactose, mannitol and crospolyvinylpyrrolidone were weighed according to the formula amount and mixed evenly; dough mess was prepared using 95% ethanol as wetting agent, granulated through 1-mesh screen, and the wet granules were dried under a condition of 50∼80°C; granule sizing was performed using 20-mesh screen, then magnesium stearate and micro powder silica gel were added and mixed evenly, compressed, to obtain mangiferin aglycone dispersible tablet of the present invention.

A mangiferin aglycone crystal form I and the preparation method thereof proposed by the present invention have been described by Examples, and the mangiferin aglycone crystal form I and the preparation method thereof can be modified or properly altered and combined by a skilled in the art to achieve the technology of the present invention, without departing from the content, spirit and scope of the present invention. Specifically, all the similar alternatives and alterations are obvious to a skilled in the art, which are deemed to be within the spirit, scope and content of the present invention.

## Claims

1. A mangiferin aglycone crystal form I, **characterized in that**, the characteristic peaks of X-ray powder diffraction spectra represented by 2θ angle using Cu-Kα radiation with λ=1.5405A are as follows:
| 2θ | I/I₀% | 2θ | I/I₀% |
|---|---|---|---|
| 7.32 | 58 | 19.18 | 11 |
| 7.80 | 24 | 25.56 | 16 |
| 9.96 | 19 | 26.42 | 22 |
| 12.46 | 100 | 27.58 | 13 |
| 13.06 | 30 | 28.58 | 19 |
| 15.72 | 16 | | |

2. The mangiferin aglycone crystal form I according to claim 1, **characterized in that**, the infrared absorption spectra thereof have characteristic absorption peaks at wave numbers: 3347±2, 3146±2, 1652±2, 1616±2, 1506±2, 1418±2, 1373±2, 1304±2, 1225±2, 1127±2, 800±2, 754±2, 732±2, 706±2, 664±2, 619±2, 570±2, 542±2 and 447±2 cm⁻¹.

3. The mangiferin aglycone crystal form I according to claim 1, **characterized in that**, the DSC analysis thereof shows a thermal transition temperature of 384.7∼388.4°C, or
**characterized in that**, the melting decomposition temperature thereof is 379.1∼403.0°C; or
**characterized in that**, the TGA analysis thereof shows a melting decomposition accompanied with a mass attenuation of 31%∼34%.

4. A method for preparing the mangiferin aglycone crystal form I according to claim 1, comprising the following steps:
Step 1: A solvent is added to mangiferin aglycone to dissolve it thoroughly, wherein the solvent is a mixture of water and aliphatic alcohol having 1∼4 carbon atoms, or a mixture of water and aliphatic ketone having 1∼4 carbon atoms;
Step 2: 0%∼5% activated charcoal and 0%∼10% acetic acid are added based on the mass of mangiferin aglycone, and filtered;
Step 3: The filtrate is allowed to stand, and crystal is slowly precipitated, to obtain the mangiferin aglycone crystal form I.

5. The preparation method according to claim 4, **characterized in that**, the volume ratio between the aliphatic alcohol having 1∼4 carbon atoms or the aliphatic ketone having 1∼4 carbon atoms and water in the solvent of step 1 is 1:0.01∼1:100; or
**characterized in that**, the mass/volume ratio in g/ml between the mangiferin aglycone and the solvent in step 1 is 1:10∼200; or
**characterized in that**, solvent added in step 1 is heated to 50∼100 °C; or **characterized in that**, the temperature for the standing in step 3 is -5∼40°C and kept for 1∼72 hours.

6. A pharmaceutical composition, comprising effective amount of the mangiferin aglycone crystal form I according to claim 1 and pharmaceutically acceptable carrier.

7. A freeze-dried powder for injection of the pharmaceutical composition according to claim 6, **characterized in that**, the freeze-dried powder for injection is consisted by weight of:
| | |
|---|---|
| mangiferin aglycone crystal form I | 1 ∼ 100mg, |
| cosolvent | 1 ∼ 200mg, and |
| excipient | 2 ∼ 1000mg. |

8. The freeze-dried powder for injection according to claim 7, **characterized in that**, the cosolvent is one of water-soluble cyclodextrin or alkaline respectively, wherein the water-soluble cyclodextrin is preferably hydroxypropyl-β-cyclodextrin or methyl-β-cyclodextrin, and the alkaline is preferably sodium bicarbonate, sodium carbonate, sodium hydroxide, arginine, lysine or meglumine; the excipient is one selected from the group consisting of mannitol, low molecular dextran, sodium chloride, sucrose and glucose, or any combination thereof.

9. A soft capsule of the pharmaceutical composition according to claim 6, **characterized in that**, the capsule content is consisted of:
| | |
|---|---|
| mangiferin aglycone crystal form I | 1 mg ∼ 100 mg, and |
| dispersing agent | 100 mg ∼ 1000 mg. |

10. The capsule according to claim 9, **characterized in that**, the dispersing agent is any one of soybean oil, corn oil, peanut oil, linseed oil, fish oil, evening primrose oil, α-linolenic acid, γ-linolenic acid, docosahexaenoic acid and eicosapentaenoic acid.

11. The capsule according to claim 9 or 10, **characterized in that**, the capsule shell is consisted of:
| | |
|---|---|
| geletin | 60 mg, |
| glycerol | 20 mg, and |
| ethyl p-hydroxybenzoate | 0.01 mg ∼ 0.3 mg. |

12. A sustained tablet of the pharmaceutical composition according to claim 6, **characterized in that**, the sustained tablet is consisted of:
| | |
|---|---|
| mangiferin aglycone crystal form I | 1 mg ∼ 100 mg, |
| skeleton agent | 10 mg ∼ 1000 mg, |
| filler | 30 mg ∼ 2000 mg, and |
| magnesium stearate | 1 mg ∼ 5 mg. |

13. The sustained tablet according to claim 12, **characterized in that** the skeleton agent is one selected from the group consisting of hydroxypropyl methyl cellulose, stearic acid, ethyl cellulose and beewax, or any combination thereof; the filler is one selected from the group consisting of lactose, pregelatinized starch, microcrystalline cellulose and micro powder silica gel, or any combination thereof.

14. A dispersible tablet of the pharmaceutical composition according to claim 6, **characterized in that**, the dispersible tablet is consisted of:
| | |
|---|---|
| mangiferin aglycone crystal form I | 1 mg ∼ 100 mg, |
| disintegrating agent | 10 mg ∼ 1000 mg, |
| filler | 30 mg ∼ 2000 mg, and |
| magnesium stearate | 1 mg ∼ 5 mg. |

15. The dispersible tablet according to claim 14, **characterized in that**, the disintegrating agent is any one selected from lactose and mannitol, or a mixture thereof; the filler is one selected from the group consisting of microcrystalline cellulose, crosslinked polyvinylpyrrolidone, polyvidone and micro powder silica gel, or any combination thereof.

## Patentansprüche

1. Mangiferin-Aglykon-Kristallform I, **dadurch gekennzeichnet, dass** die charakteristischen Spitzen von Röntgenpulverdiffraktionsspektren dargestellt durch 2θ Winkel mithilfe von Cu-Ka-Strahlung mit λ = 1,5405A wie folgt sind:
| 2θ | 1/1₀% | 20 | 1/1₀% |
|---|---|---|---|
| 7,32 | 58 | 19,18 | 11 |
| 7,80 | 24 | 25,56 | 16 |
| 9,96 | 19 | 26,42 | 22 |
| 12,46 | 100 | 27,58 | 13 |
| 13,06 | 30 | 28,58 | 19 |
| 15,72 | 16 | | |

2. Mangiferin-Aglykon-Kristallform I nach Anspruch 1, **dadurch gekennzeichnet, dass** die Infrarotabsorptionsspektren derselben charakteristische Absorptionsspitzen bei den Wellenzahlen: 3347±2, 3146±2, 1652±2, 1616±2, 1506±2, 1418±2, 1373±2, 1304±2, 1225±2, 1127±2, 800±2, 754±2, 732±2, 706±2, 664±2, 619±2, 570±2, 542±2 und 447±2 cm⁻¹ aufweisen.

3. Mangiferin-Aglykon-Kristallform I nach Anspruch 1, **dadurch gekennzeichnet, dass** die DSC-Analyse derselben eine Wärmeübergangstemperatur von 384,7∼388,4°C zeigt oder
**dadurch gekennzeichnet, dass** die Schmelzzersetzungstemperatur derselben 379,1∼4030°C beträgt; oder
**dadurch gekennzeichnet, dass** die TGA-Analyse derselben eine Schmelzzersetzung begleitet von einer Massendämpfung von 31%∼34% zeigt.

4. Verfahren zum Herstellen der Mangiferin-Aglykon-Kristallform I nach Anspruch 1, welches die folgenden Schritte umfasst:
Schritt 1: Mangiferin-Aglykon wird ein Lösungsmittel zugegeben, um es gründlich aufzulösen, wobei das Lösungsmittel ein Gemisch aus Wasser und aliphatischem Alkohol mit 1∼4 Kohlenstoffatomen oder ein Gemisch aus Wasser und aliphatischem Keton mit 1∼4 Kohlenstoffatomen ist;
Schritt 2: Es werden 0%∼5% Aktivkohle und 0%∼10% Essigsäure beruhend auf der Masse von Mangiferin-Aglykon zugegeben und filtriert;
Schritt 3: Das Filtrat wird stehen gelassen und Kristall wird langsam ausgefällt, um die Mangiferin-Aglykon-Kristallform I zu erhalten.

5. Herstellungsverfahren nach Anspruch 4, **dadurch gekennzeichnet, dass** das Volumenverhältnis zwischen dem aliphatischen Alkohol mit 1∼4 Kohlenstoffatomen oder dem aliphatischen Keton mit 1∼4 Kohlenstoffatomen und Wasser in dem Lösungsmittel von Schritt 1 1:0,01∼1:100 beträgt; oder
**dadurch gekennzeichnet, dass** das Masse/Volumen-Verhältnis in g/ml zwischen dem Mangiferin-Aglykon und dem Lösungsmittel in Schritt 1 1:10∼200 beträgt; oder
**dadurch gekennzeichnet, dass** in Schritt 1 zugegebenes Lösungsmittel auf 50∼100 °C erwärmt wird; oder
**dadurch gekennzeichnet, dass** die Temperatur für das Stehenlassen in Schritt 3 -5∼40°C beträgt und 1∼72 Stunden gehalten wird.

6. Pharmazeutische Zusammensetzung, welche eine Wirkmenge der Mangiferin-Aglykon-Kristallform I nach Anspruch 1 und einen pharmazeutisch zulässigen Träger umfasst.

7. Gefriergetrocknetes Pulver zur Injektion der pharmazeutischen Zusammensetzung nach Anspruch 6, **dadurch gekennzeichnet, dass** das gefriergetrocknete Pulver zur Injektion nach Gewicht besteht aus:
| | |
|---|---|
| Mangiferin-Aglykon-Kristallform I | 1 ∼ 100 mg, |
| Hilfslösungsmittel | 1 ∼ 200 mg und |
| Bindemittel | 2 ∼ 1000 mg. |

8. Gefriergetrocknetes Pulver zur Injektion nach Anspruch 7, **dadurch gekennzeichnet, dass** das Hilfslösungsmittel wasserlösliches Cyclodextrin oder alkalisch ist, wobei das wasserlösliche Cyclodextrin bevorzugt Hydroxypropyl-β-cyclodextrin oder Methyl-β-cyclodextrin ist und der alkalische Stoff bevorzugt Natriumbicarbonat, Natriumcarbonat, Natriumhydroxid, Arginin, Lysin oder Meglumin ist; das Bindemittel eines gewählt aus der Gruppe bestehend aus Mannitol, niedermolekularem Dextran, Natriumchlorid, Saccharose und Glukose oder eine beliebige Kombination derselben ist.

9. Weichkapsel der pharmazeutischen Zusammensetzung nach Anspruch 1, **dadurch gekennzeichnet, dass** der Kapselinhalt besteht aus:
| | |
|---|---|
| Mangiferin-Aglykon-Kristallform I | 1 mg ∼ 100 mg und |
| Dispergiermittel | 100 mg ∼ 1000 mg. |

10. Kapsel nach Anspruch 9, **dadurch gekennzeichnet, dass** das Dispergiermittel eines von Sojabohnenöl, Maisöl, Erdnussöl, Leinsamenöl, Fischöl, Nachtkerzenöl, α-Linolensäure, γ-Linolensäure, Docosahexaensäure und Eicosapentaensäure ist.

11. Kapsel nach Anspruch 9 oder 10, **dadurch gekennzeichnet, dass** die Kapselhülle besteht aus:
| | |
|---|---|
| Gelatine | 60 mg, |
| Glycerol | 20 mg und |
| Ethyl-p-hydroxybenzoat | 0,01 mg ∼ 0,3 mg. |

12. Retard-Tablette der pharmazeutischen Zusammensetzung nach Anspruch 6, **dadurch gekennzeichnet, dass** die Retard-Tablette besteht aus:
| | |
|---|---|
| Mangiferin-Aglykon-Kristallform I | 1 mg ∼ 100 mg, |
| Gerüst-Agens | 10 mg ∼ 1000 mg, |
| Füllstoff | 30 mg ∼ 2000 mg und |
| Magnesiumstearat | 1 mg ∼ 5 mg. |

13. Retard-Tablette nach Anspruch 12, **dadurch gekennzeichnet, dass** das Gerüst-Agens eines gewählt aus der Gruppe bestehend aus Hydroxypropylmethylcellulose, Stearinsäure, Ethylcellulose und Bienenwachs oder eine beliebige Kombination derselben ist; der Füllstoff einer gewählt aus der Gruppe bestehend aus Laktose, Quellstärke, mikrokristalliner Cellulose und Mikropulver-Silikagel oder eine beliebige Kombination derselben ist.

14. Dispergierbare Tablette der pharmazeutischen Zusammensetzung nach Anspruch 6, **dadurch gekennzeichnet, dass** die dispergierbare Tablette besteht aus:
| | |
|---|---|
| Mangiferin-Aglykon-Kristallform I | 1 mg ∼ 100 mg, |
| Aufschlussmittel | 10 mg ∼ 1000 mg, |
| Füllstoff | 30 mg ∼ 2000 mg und |
| Magnesiumstearat | 1 mg ∼ 5 mg. |

15. Dispergierbare Tablette nach Anspruch 14, **dadurch gekennzeichnet, dass** das Aufschlussmittel ein beliebiges gewählt aus Laktose und Mannitol oder eine Mischung derselben ist; der Füllstoff einer gewählt aus der Gruppe bestehend aus mikrokristalliner Cellulose, vernetztem Polyvinylpyrrolidon, Polyvidon und Mikropulver-Silikagel oder eine beliebige Kombination derselben ist.

## Revendications

1. Forme cristalline I de l'aglycone de mangiférine, **caractérisée en ce que**,
les pics caractéristiques de spectres de diffraction X sur poudre représentés par l'angle 2θ en utilisant un rayonnement Cu-Kα avec λ= 1,5405 A sont comme suit :
| 2θ | I/I₀% | 20 | I/I₀% |
|---|---|---|---|
| 7,32 | 58 | 19,18 | 11 |
| 7,80 | 24 | 25,56 | 16 |
| 9,96 | 19 | 26,42 | 22 |
| 12,46 | 100 | 27,58 | 13 |
| 13,06 | 30 | 28,58 | 19 |
| 15,72 | 16 | | |

2. Forme cristalline I de l'aglycone de mangiférine selon la revendication 1, **caractérisée en ce que**, les spectres d'absorption infrarouge de celle-ci ont des pics d'absorption caractéristiques aux nombres d'onde : 3347 ± 2, 3146 ± 2, 1652 ± 2, 1616 ± 2, 1506 ± 2, 1418 ± 2, 1373 ± 2, 1304 ± 2, 1225 ± 2, 1127 ± 2, 800 ± 2, 754 ± 2, 732 ± 2, 706 ± 2, 664 ± 2, 619 ± 2, 570 ± 2, 542 ± 2 et 447 ± 2 cm⁻¹.

3. Forme cristalline I de l'aglycone de mangiférine selon la revendication 1, **caractérisée en ce que**, l'analyse calorimétrique différentielle (DSC) de celle-ci montre une température de transition thermique de 384,7 à 388,4 °C, ou **caractérisée en ce que**, la température de décomposition par fusion de celle-ci est de 379,1 à 403,0 °C ; ou **caractérisée en ce que**, l'analyse thermogravimétrique (TGA) de celle-ci montre une décomposition par fusion accompagnée d'une baisse de masse de 31 % à 34 %.

4. Procédé de fabrication de la forme cristalline I de l'aglycone de mangiférine selon la revendication 1, comprenant les étapes suivantes :
Étape 1 : un solvant est ajouté à l'aglycone de mangiférine afin de le dissoudre complètement, le solvant étant un mélange of eau et d'alcool aliphatique ayant de 1 à 4 atomes de carbone, ou un mélange d'eau et de cétone aliphatique ayant de 1 à 4 atomes de carbone ;
Étape 2 : de 0 % à 5 % de charbon activé et 0 % à 10 % d'acide acétique sont ajoutés sur la base de la masse d'aglycone de mangiférine, et filtrés ;
Étape 3 : on laisse reposer le filtrat et on fait lentement précipiter le cristal, afin d'obtenir la forme cristalline I de l'aglycone de mangiférine.

5. Procédé de fabrication selon la revendication 4, **caractérisé en ce que**, le rapport volumique entre l'alcool aliphatique ayant de 1 à 4 atomes de carbone ou la cétone aliphatique ayant de 1 à 4 atomes de carbone et l'eau dans le solvant de l'étape 1 va de 1/0,01 à 1/100 ; ou **caractérisé en ce que**, le rapport masse/volume en g/ml entre l'aglycone de mangiférine et le solvant dans l'étape 1 va de 1/10 à 200 ; ou **caractérisé en ce que**, le solvant ajouté à l'étape 1 est chauffé de 50 à 100 °C ; ou **caractérisé en ce que**, la température pendant le repos de l'étape 3 va de -5 à 40 °C et elle est maintenue pendant 1 à 72 heures.

6. Composition pharmaceutique, comprenant une quantité efficace de la forme cristalline I de l'aglycone de mangiférine selon la revendication 1 et un véhicule pharmaceutiquement acceptable.

7. Poudre lyophilisée pour injection de la composition pharmaceutique selon la revendication 6, **caractérisée en ce que**, la poudre lyophilisée pour injection est constituée en poids de :
| | |
|---|---|
| forme cristalline I de l'aglycone de mangiférine | 1 à 100 mg, |
| co-solvant | 1 à 200 mg et |
| excipient | 2 à 1000 mg. |

8. Poudre lyophilisée pour injection selon la revendication 7, **caractérisée en ce que**,
le co-solvant est l'un respectivement parmi une cyclodextrine hydrosoluble ou un alcalin, où
la cyclodextrine hydrosoluble est de préférence l'hydroxypropyl-β-cyclodextrine ou la méthyl-β-cyclodextrine, et l'alcalin est de préférence le bicarbonate de sodium, le carbonate de sodium, l'hydroxyde de sodium, l'arginine, la lysine ou la méglumine ; l'excipient est choisi dans le groupe constitué de mannitol, dextrane de faible masse moléculaire, chlorure de sodium, sucrose et glucose, ou d'une quelconque combinaison de ceux-ci.

9. Gélule molle de la composition pharmaceutique selon la revendication 6, **caractérisée en ce que**, le contenu de la gélule est constitué de :
| | |
|---|---|
| forme cristalline I de l'aglycone de mangiférine | 1 mg à 100 mg et |
| agent dispersant | 100 mg à 1000 mg. |

10. Gélule selon la revendication 9, **caractérisée en ce que**, l'agent dispersant est l'un quelconque parmi l'huile de soja, l'huile de maïs, l'huile de graine de lin, l'huile de poisson, l'huile d'onagre, l'acide α-linolénique, l'acide γ-linolénique, l'acide docosahexaénoïque et l'acide éicosapentaénoïque.

11. Gélule selon la revendication 9 ou 10, **caractérisée en ce que**, l'enveloppe de la gélule est constituée de :
| | |
|---|---|
| gélatine | 60 mg, |
| glycérol | 20 mg et |
| p-hydroxybenzoate d'éthyle | 0,01 mg à 0,3 mg. |

12. Comprimé à libération prolongée de la composition pharmaceutique selon la revendication 6, **caractérisé en ce que**, le comprimé à libération prolongée est constitué de :
| | |
|---|---|
| forme cristalline I de l'aglycone de mangiférine | 1 à 100 mg |
| agent de structure | 10 mg à 1000 mg, |
| charge | 30 mg à 2000 mg et |
| stéarate de magnésium | 1 mg à 5 mg. |

13. Comprimé à libération prolongée selon la revendication 12, **caractérisé en ce que**, l'agent de structure est choisi dans le groupe constitué d'hydroxypropylméthylcellulose, acide stéarique, éthylcellulose et cire d'abeille, ou n'importe quelle combinaison de ceux-ci ; la charge est choisie dans le groupe constitué de lactose, amidon prégélatinisé, cellulose microcristalline et gel de silice en micropoudre, ou n'importe quelle combinaison de ceux-ci.

14. Comprimé dispersible de la composition pharmaceutique selon la revendication 6, **caractérisé en ce que**, le comprimé dispersible est constitué de :
| | |
|---|---|
| forme cristalline I de l'aglycone de mangiférine | 1 mg à 100 mg, |
| agent de désintégration | 10 mg à 1000 mg, |
| charge | 30 mg à 2000 mg et |
| stéarate de magnésium | 1 mg à 5 mg. |

15. Comprimé dispersible selon la revendication 14, **caractérisé en ce que**, l'agent de désintégration est l'un quelconque parmi le lactose et le mannitol, ou un mélange de ceux-ci ; la charge est choisie dans le groupe constitué de cellulose microcristalline, polyvinylpyrrolidone réticulée, polyvidone et gel de silice en micropoudre, ou n'importe quelle combinaison de ceux-ci.
